# EUROPEAN PATENT SPECIFICATION

(11) **EP 3 493 849 B1**
(45) Date of publication and mention of the grant of the patent: **01.12.2021**
(21) Application number: 17793720.8
(22) Date of filing: 04.08.2017
(51) Int. Cl.: A61K 31/166, A61K 31/167, A61K 45/06, A61P 1/16

(54) **NEW USE OF N,N-BIS-2-MERCAPTOETHYL ISOPHTHALAMIDE**
NEUE VERWENDUNG VON N,N-BIS-MERCAPTOETHYLISOPHTHALAMID
NOUVELLE UTILISATION DU N, N-BIS-2-MERCAPTOÉTHYLE ISOPHTALAMIDE

(30) Priority: 05.08.2016 GB 201613535
(43) Date of publication of application: 12.06.2019
(73) Proprietor: EmeraMed Limited, Dublin 6 (IE)
(72) Inventor: HALEY, Boyd Eugene, Nicholasville Kentucky 40356 (US); KLINGBERG, Ragnar Axel Theodor, Stockholm 115 35 (SE)
(74) Representative: Potter Clarkson
(86) International application number: PCT/GB2017/052306
(87) International publication number: WO 2018/025049

(56) References cited:
- WO-A2-2011/006097
- US-A1- 2010 227 812
- KHAYYAT AHDAB ET AL: "Contribution of N-Acetylcysteineamide as a New Thiol-Antioxidant in the Treatment of Acetaminophen Toxicity", FREE RADICAL BIOLOGY AND MEDICINE, vol. 87, October 2015 (2015-10), XP029334172, ISSN: 0891-5849, DOI: 10.1016/J.FREERADBIOMED.2015.10.240
- RISHI B. PATEL ET AL: "Thiol-redox antioxidants protect against lung vascular endothelial cytoskeletal alterations caused by pulmonary fibrosis inducer, bleomycin: comparison between classical thiol-protectant, N -acetyl-l-cysteine, and novel thiol antioxidant, N,N' -bis-2-mercaptoethyl isophthalamide", TOXICOLOGY MECHANISMS AND METHODS, vol. 22, no. 5, 1 June 2012 (2012-06-01), pages 383-396, XP055199056, ISSN: 1537-6516, DOI: 10.3109/15376516.2012.673089
- "Treatment of acute paracetamol poisoning", BMJ. BRITISH MEDICAL JOURNAL, LONDON, GB, vol. 2, no. 6085, 20 August 1977 (1977-08-20), pages 481-482, XP009502006, ISSN: 0959-8146, DOI: 10.1136/BMJ.2.6085.481

## Description

### Field of the Invention

The present invention relates to new medical uses of the compound *N,N-bis*-2-mercaptoethyl isophthalamide (NBMI) and pharmaceutically acceptable salts thereof. In particular, the invention relates to the use of such compounds in preventing the effects of paracetamol toxicity, which may occur due to the toxic effects of paracetamol overdose. More particularly, the invention may relate to the prevention of acute liver failure occurring as a result of paracetamol toxicity.

### Background of the Invention

The listing or discussion of an apparently prior-published document in this specification should not necessarily be taken as an acknowledgement that the document is part of the state of the art or is common general knowledge.

Liver disorders caused by damage to the liver represent serious and often life-threatening conditions. Acute liver failure (ALF) is among the most critical of liver disorders, with a very high incidence of mortality among sufferers. There are many potential causes of ALF, including the progression of liver diseases, such as viral hepatitis, alcoholic liver disease and non-alcoholic fatty liver disease, and the toxic effects of medication.

Among those medications known to be potential causes of liver damage leading to ALF, paracetamol toxicity stands out as being particularly dangerous, not least due to its potential to cause rapid damage to liver tissue, often leading to irreparable damage to the liver and thus to irreversible liver failure.

Paracetamol (also known as acetaminophen) is a widely used and effective pain killer for mild to moderate pain. However, as it is highly toxic at doses only moderately greater than the effective therapeutic dose, accidental paracetamol intoxication is a significant risk. Moreover, as it is commonly known to have harmful effects when administered in excess, deliberate paracetamol intoxication through overdose has been used as a means for self-harm in those with suicidal tendencies. For these reasons, paracetamol overdose is the most common form of intoxication from authorised medication, leading to over 100,000 emergency room visits per year in the US and the EU alone.

Following administration, paracetamol is metabolised in the liver to the toxic intermediate *N*-acetyl-*p*-benzoquinone imine (NAPQI), which under normal circumstances is detoxified through conjugation with the endogenous antioxidant glutathione and consequently excreted as the non-toxic cysteinyl paracetamol. However, in times of stress, such as in the event of a paracetamol overdose, stores of glutathione in the liver are depleted, leading to oxidative stress and the formation of NAPQI-protein adducts, which in turn causes rapid liver damage. In serious cases, this toxic effect leads to ALF and, if left untreated, may result in death.

The standard treatment for paracetamol toxicity is to administer high doses of *N*-acetyl cysteine (NAC) over an extended period. NAC is an analogue of cysteine which is a precursor to glutathione and thus is thought to replenish stores of glutathione in the liver, in turn leading to the restoration of its protective effect. Treatment of paracetamol toxicity with NAC is effective when it is administered within 8 hours of the ingestion of a toxic dose of paracetamol. However, the effectiveness of NAC treatment rapidly diminishes if it is administered more than 8 hours after paracetamol ingestion, as the glutathione levels have then decreased too much (approximately below 70% of normal levels) and cannot bind to all of the NAPQI metabolite, which then causes damage to the liver. While NAC itself does bind to NAPQI *in vitro,* NAC is unable to halt an intoxication *in vivo.*

WO 2011/006097 describes the administration of prostaglandin E2 (PGE2) in combination with NAC for the treatment of paracetamol toxicity.

Therefore there is a significant clinically-unmet need for the development of novel and effective alternative therapies for the treatment and prevention of liver damage caused by paracetamol toxicity.

*N*,*N*-*bis*-2-mercaptoethyl isophthalamide (NBMI) was first disclosed in a patent application granted as US patent number US 6,586,600 B2. Its use as a dietary supplement is disclosed in US patent application 2010/0227812, and it is also known to be a chelator of heavy metals, such as mercury, cadmium and lead. Analogues of NBMI have been disclosed in, *inter alia*, granted US patent US 8,426,368 B2, and international patent applications WO 2011/038385 and WO 2012/121798.

However, there has been no teaching or suggestion relating to the potential use of NBMI thereof in the prevention of liver damage caused by paracetamol toxicity.

### Description of the Invention

We have now surprisingly found that administration of NBMI following paracetamol overdose can prevent or reduce liver damage, thus providing an effecting means to prevent the effects of paracetamol toxicity. NBMI therefore shows great promise as an improved treatment for paracetamol toxicity and acute liver failure related to paracetamol toxicity. The invention is defined by the appended claims.

### New methods and medical uses

According to a first aspect of the invention there is provided the compound *N,N-bis*-2-mercaptoethyl isophthalamide (NBMI), or a pharmaceutically-acceptable salt thereof, for use in the prevention of paracetamol toxicity.

Unless specified otherwise, NBMI and pharmaceutically-acceptable salts thereof may be referred to herein as "compounds of the invention".

In an alternative first aspect of the invention there is provided the use of the compound *N*,*N*-*bis*-2-mercaptoethyl isophthalamide, or a pharmaceutically-acceptable salt thereof, in the manufacture of a medicament for the prevention of paracetamol toxicity.

In a further alternative first aspect of the invention there is provided the use of NBMI in a method for the prevention of paracetamol toxicity, which method comprises the administration of an effective amount of *N*,*N*-*bis*-2-mercaptoethyl isophthalamide, or a pharmaceutically-acceptable salt thereof, to a patient in need thereof (i.e. a patient of such prevention).

Unless indicated otherwise, all technical and scientific terms used herein have the same meaning as commonly understood by one of ordinary skill in the art to which this invention pertains. Any references in the description to methods for the prevention of paracetamol toxicity refer to the compounds, kits or formulations of the present invention for use in a method of preventing paracetamol toxicity.

Particular features and embodiments described in relation to a given aspect of the invention should, unless the context indicates otherwise, be regarded as having been disclosed in combination with any and all other particular features and embodiments of that aspect of the invention.

For the avoidance of doubt, the compound NBMI as described herein may also be referred to by the trade name Irminix^{®} or by the international non-proprietary name (INN) Emeramide. The structure of the compound (in non-salt form) is represented below.

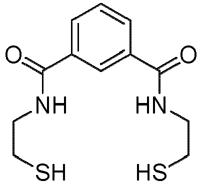

Pharmaceutically acceptable salts as referred to within the scope of the present invention include acid addition salts and base addition salts. Such salts may be formed by conventional means; for example, by reaction of a free acid or a free base form of a compound of the invention with one or more equivalents of an appropriate acid or base, optionally in a solvent, or in a medium in which the salt is insoluble, followed by removal of said solvent, or said medium, using standard techniques (e.g. under reduced pressure, by freeze-drying or by filtration). Salts may also be prepared by exchanging a counter-ion of a compound of the invention in the form of a salt with another counter-ion, for example, using a suitable ion exchange resin.

Particular acid addition salts that may be mentioned include carboxylate salts (e.g. formate, acetate, trifluoroacetate, propionate, isobutyrate, heptanoate, decanoate, caprate, caprylate, stearate, acrylate, caproate, propiolate, ascorbate, citrate, glucuronate, glutamate, glycolate, α-hydroxybutyrate, lactate, tartrate, phenylacetate, mandelate, phenylpropionate, phenylbutyrate, benzoate, chlorobenzoate, methylbenzoate, hydroxybenzoate, methoxybenzoate, dinitrobenzoate, o-acetoxy-benzoate, salicylate, nicotinate, isonicotinate, cinnamate, oxalate, malonate, succinate, suberate, sebacate, fumarate, malate, maleate, hydroxymaleate, hippurate, phthalate or terephthalate salts), halide salts (e.g. chloride, bromide or iodide salts), sulphonate salts (e.g. benzenesulphonate, methyl-, bromo- or chloro-benzenesulphonate, xylenesulphonate, methanesulphonate, ethanesulphonate, propanesulphonate, hydroxy-ethanesulphonate, 1- or 2- naphthalene-sulphonate or 1,5-naphthalenedisulphonate salts) or sulphate, pyrosulphate, bisulphate, sulphite, bisulphite, phosphate, monohydrogenphosphate, dihydrogenphosphate, metaphosphate, pyrophosphate or nitrate salts, and the like.

Particular base addition salts that may be mentioned include salts formed with alkali metals (such as Na and K salts), alkaline earth metals (such as Mg and Ca salts), organic and inorganic bases (such as aluminium hydroxide). More particularly, base addition salts that may be mentioned include Mg, Ca and, most particularly, K and Na salts.

Particular pharmaceutically-acceptable salts that may be mentioned (in particular, when forming salts of NBMI) include base addition salts, such as those formed with alkali metals (e.g. salts formed with Na or K).

The skilled person will understand that references to pharmaceutically-acceptable derivatives of NBMI will include compounds formed by derivatisation of NBMI following procedures known to those skilled in the art, such as through modification of one or more of the -NH- and/or -SH moieties therein. For example, references to such derivatives may include derivatisation of -NH- moieties through alkylation thereof (e.g. to form an -NR¹-moiety, wherein R¹ represents C₁₋₃ alkyl optionally substituted with one or more fluoro group, such as wherein R¹ represents methyl) and/or derivatisation of -SH moieties through alkylation thereof (e.g. to form an -SR² moiety, wherein R² represents C₁₋₃ alkyl optionally substituted with one or more fluoro group, such as wherein R² represents methyl) or esterification thereof (e.g. to form an -SC(O)R³ moiety, wherein R³ represents C₁₋₃ alkyl optionally substituted with one or more fluoro group, such as wherein R³ represents methyl, or another moiety resulting from reaction of a pharmaceutically-acceptable compound capable of forming such thioesters, such as a pharmaceutically-acceptable carboxylic acid or ester).

Particular pharmaceutically-acceptable derivatives of NBMI that may be mentioned include the di-sulphide bridged additions of glutathione, cysteine, alphadihydrolipoic acid, cystamine, thiolphosphate, 5'-thioladenosine, L-homocysteine, co-enzyme A, 2-mercaptoethanol and dithiothreitol,. Such derivatives may be prepared by analogy to the procedures described in, for example, US patent application 2011/0237776.

For the avoidance of doubt, references to "*N*,*N*-*bis*-2-mercaptoethyl isophthalamide (NBMI), or a pharmaceutically-acceptable salt thereof" will indicate that NBMI may be present in the form of a pharmaceutically-acceptable salt thereof.

Particular compounds of the invention that may be mentioned include NBMI and pharmaceutically-acceptable salts thereof.

More particular compounds of the invention that may be mentioned include NBMI.

For the avoidance of doubt, compounds of the invention may exist as solids, and thus the scope of the invention includes all amorphous, crystalline and part crystalline forms thereof. Where compounds of the invention exist in crystalline and part crystalline forms, such forms may include solvates, which are included in the scope of the invention. Compounds of the invention may also exist in solution.

The present invention also embraces isotopically-labelled compounds of the invention, which are identical, but for the fact that one or more atoms are replaced by an atom having an atomic mass or mass number different from the atomic mass or mass number usually found in nature (or the most abundant one found in nature). All isotopes of any particular atom or element as specified herein are contemplated within the scope of the compounds of the invention. Hence, references to the compounds of the invention also includes deuterated compounds, i.e. in which one or more hydrogen atoms are replaced by the hydrogen isotope deuterium.

The skilled person will understand that references herein to "prevention" of a particular condition (and, similarly, to "preventing" that condition) take their normal meanings in the art. In particular, these terms may refer to achieving a reduction in the likelihood of developing the relevant condition or symptoms associated with the relevant condition (for example, a reduction of at least 10% when compared to the baseline level, such as a reduction of at least 20% or, more particularly, a reduction of at least 30%). Similarly, the term "preventing" may also be referred to as "prophylaxis" of the relevant condition, and vice versa.

For example, when used in relation to paracetamol toxicity, references to "prevention" may refer to reducing the likelihood that the patient will experience the effects of paracetamol toxicity, such as liver damage (including ALF). Similarly, references to "treating" may refer to reducing the severity of the effects of paracetamol toxicity, such as liver damage (including ALF).

As used herein, references to "patients" will refer to a living subject being treated, including mammalian (in particular, human) patients, and as such "patients" may also be referred to as "subjects", and *vice versa.* References to "patients" (and therefore also to "subjects") also should be considered to refer to individuals displaying no symptoms of the relevant condition, for whom compounds of the invention may be used as a preventative or prophylactic measure (as defined herein above).

For the avoidance of doubt, references to patients may also include references to animals, such as non-mammalian animals (e.g. birds) and, particularly, mammalian animals (e.g. cats, dogs, rabbits, rodents, horses, sheep, pigs, goats, cows, primates, and the like).

As used herein, the term "effective amount" will refer to an amount of a compound that confers the desired therapeutic effect on the treated subject (i.e. the desired prevention, as described herein). The effect may be objective (i.e. measurable by some test or marker) or subjective (i.e. the subject gives an indication of and/or feels an effect).

The skilled person will understand that paracetamol toxicity (and similarly paracetamol intoxication) refers to a condition characterised by the clinical effects experienced following administration of a toxic dose (i.e. an overdose) of paracetamol. As such, the prevention of paracetamol toxicity may also be referred to as the treatment of paracetamol overdose (which may be deliberate or accidental), paracetamol abuse (which may refer to deliberate paracetamol overdose), paracetamol poisoning (which may be deliberate or accidental) and the like.

In particular, paracetamol toxicity may be indicated by serum liver enzyme alanine transaminase (ALT) and/or aminotransferase (AST) levels above 1000 Unit/L. It may be characterised by hepatocellular necrosis (i.e. damage to the cells of the liver), which may in turn be characterised by impaired liver function and, in severe cases, acute liver failure (ALF). Such effects may initially (i.e. in the first 24 hours following paracetamol overdose) be characterised by symptoms such as nausea and vomiting, followed by the onset of right subcostal pain and tenderness, indicating the onset of liver damage.

The skilled person will understand that the amount constituting a toxic dose of paracetamol is highly variable and may depend on a number of factors, such as the weight, age and general health of the patient. Toxic doses of paracetamol may occur through acute overdose (i.e. an overdose occurring as a resulting of a toxic amount being administered in any one dose) or chronic dose (i.e. an overdose occurring as a result of the cumulative effect of several doses). In general, cumulative daily doses above about 10 to 12 g (or about 150 mg/kg of body weight) and single doses about 4.5 to 6 g (or 75 mg/kg of body weight) are generally believed to be likely to induce toxicity.

Moreover, the skilled person will understand that paracetamol toxicity may occur at lower doses in patients with greater susceptibility to such toxicity, such as those experiencing impaired liver function (i.e. those experiencing impaired liver function independently of the immediate effects of paracetamol toxicity). Such impaired liver function may be the result of malnutrition or fasting, a chronic condition (such as viral infection, e.g. hepatitis infection, or liver damage caused by chronic alcoholism) or an acute condition (such as impaired liver function caused by the effects of medication, i.e. medication other than paracetamol, or excessive alcohol consumption).

In any event, the skilled person will appreciate that the prevention of paracetamol toxicity may be performed in patients known to have (or, in circumstances when a cautious approach is required, subjects thought to have) received (e.g. through ingestion) any greater than therapeutic dose of paracetamol, with reference to the standard therapeutic dose for any given patient as known to those skilled in the art (for example, a dose in adults of healthy weight of 1 g every 4 hours with a maximum of 4 g per 24 hour period).

The skilled person will understand that paracetamol overdose may occur as a result of administration of paracetamol perse (e.g. in the form of paracetamol tablets, such as those typically formulated for oral administration, or via intravenous infusion) or in the form of a pharmaceutical formulation comprising paracetamol together with other active ingredients (such as formulations designed for the relief of the symptoms of common cold and influenza).

The skilled person will understand that the prevention of paracetamol toxicity as described herein should ideally be performed (i.e. should be commenced) as soon as possible after paracetamol overdose, such as within 72 hours (or, particularly, within 24 hours, such as within 12 hours or, more particularly, within 8 hours) of the overdose occurring. However, treatment with compounds of the invention may also be effective in patients who have not been treated (for paracetamol overdose) within the initial period (e.g. the initial 24 hours or, more particularly, the initial 12 hours, such as with the initial 8 hours) following overdose, in which case the treatment may be performed in a period between 168 hours and 12 hours (e.g. between 72 hours and 12 hours, such as between 72 hours and 24 hours) after paracetamol overdose. For example, treatment with compounds of the invention may be performed in patients who have not been treated (for paracetamol overdose) within the initial 8 hours following overdose.

As described herein, paracetamol toxicity may be characterised by acute liver failure. Thus, the prevention of paracetamol toxicity as described herein may be performed in patients having or at risk of developing (e.g. patients at risk of developing) acute liver failure.

Further, according to a second aspect of the invention there is provided the compound *N*,*N*-*bis*-2-mercaptoethyl isophthalamide (NBMI), or a pharmaceutically-acceptable salt thereof, for use in the prevention of acute liver failure associated with paracetamol toxicity.

In an alternative second aspect of the invention there is provided the use of the compound, *N*,*N*-*bis*-2-mercaptoethyl isophthalamide, or a pharmaceutically-acceptable salt thereof, in the manufacture of a medicament for the prevention of acute liver failure associated with paracetamol toxicity.

In a further alternative second aspect of the invention there is provided the use of NBMI in a method for the prevention of acute liver failure associated with paracetamol toxicity, which method comprises the administration of an effective amount of *N,N-bis*-2-mercaptoethyl isophthalamide, or a pharmaceutically-acceptable salt thereof, to a patient in need thereof (i.e. of such prevention).

For the avoidance of doubt, all embodiments and particular features described in relation to the first aspect of the invention (and combinations thereof) will also apply to the second aspect of the invention.

As used herein, references to "acute liver failure associated with paracetamol toxicity" will be understood to refer to acute liver failure that is triggered by (or caused by) paracetamol toxicity, which may be referred to as acute liver failure resulting from paracetamol toxicity.

The skilled person will understand that acute liver failure may be defined as the rapid development of hepatocellular dysfunction, characterised by the development of coagulopathy (typically with an international normalized ratio (INR) of greater than 1.5) and encephalopathy (typically characterised by mental status changes) in a patient without prior liver disease (e.g. without known prior liver disease). Further, it is generally understood that liver damage leading to acute liver failure may involve a loss of function in about 80% to about 90% of liver cells.

The skilled person will understand that impaired (and, similarly, reduced or abnormal) liver function refers to problematic sub-optimal liver function as determined by a clinician using techniques known to those skilled in the art. For example, common tests for impaired liver function (commonly referred to as liver function tests (LFTs)) include those that measure the levels of alanine aminotransferase (ALT), aspartate aminotransferase (AST), alkaline phosphatase (ALP), gamma glutamyltransferase (GGT), bilirubin or albumin present in a patient's blood. Similarly, sub-optimal liver function may be identified through clotting studies (such as by assessing prothrombin time (PT) or international normalized ratio (INR)). The relevant results of these tests that are indicative of impaired or abnormal liver function are well known to the medical practitioner (or other persons skilled in the field).

The skilled person will understand that the prevention of paracetamol toxicity or acute liver failure associated with paracetamol toxicity may be combined with (i.e. administered as part of the same medical intervention as) other treatments (i.e. medications and/or therapeutic methods) used for such purposes.

For example, in instances of overdose resulting from ingestion of paracetamol, prevention of paracetamol toxicity or acute liver failure associated with paracetamol toxicity may be combined with oral administration of activated charcoal or similar preparations.

Further, such prevention may be combined with administration (e.g. concomitant or sequential administration) of one or more other therapeutic agent useful in the treatment or prevention of paracetamol toxicity or acute liver failure associated with paracetamol toxicity as known to those skilled in the art, such as those described herein below.

As used herein, references to "sequential administration" may refer to separate administration of the therapeutic agents as part of the same medical intervention (e.g. within four hours, such as within two hours or, particularly within one hour, of each other).

As described herein, compounds of the invention may be particularly effective in preventing paracetamol toxicity or acute liver failure associated with paracetamol toxicity.

Thus, prevention of paracetamol toxicity or acute liver failure associated with paracetamol toxicity (as described in the first and second aspects of the invention, respectively) may be combined with treatment with paracetamol (e.g. the treatment of mild to moderate pain, fever or other similar conditions, such as through the administration of a therapeutically effective amount of paracetamol to a patient in need thereof, which treatment may be performed in a manner as known to those skilled in the art), or vice versa.

Such combinations (i.e. with paracetamol) may be particularly useful when administered to patients with greater susceptibility to paracetamol toxicity, such as those described herein, and/or in patients receiving or requiring treatment with greater doses of paracetamol than those normally used (e.g. doses greater than 1 g every 4 hours with a maximum of 4 g per 24 hour period).

### Pharmaceutical compositions and dosages

The skilled person will understand that, when employed in the uses and methods described herein, compounds of the invention may be administered in a manner allowing for systemic absorption, which absorption may occur *via* a number of possible routes; for example, compounds of the invention may be administered orally, intravenously or intraarterially, intramuscularly, cutaneously, subcutaneously, transmucosally (e.g. sublingually or buccally), rectally, transdermally, nasally, pulmonarily (e.g. by inhalation, tracheally or bronchially), or by any other parenteral route, in the form of a pharmaceutical preparation comprising the compound in a pharmaceutically acceptable dosage form. In particular, compounds of the invention may be administered orally, rectally or intravenously (e.g. by intravenous infusion).

The compounds of the invention will generally be administered in the form of one or more pharmaceutical formulations in admixture with a pharmaceutically acceptable excipient, which may be selected with due regard to the intended route of administration and standard pharmaceutical practice. Such pharmaceutically acceptable excipients may be chemically inert to the active compounds and may have no detrimental side effects or toxicity under the conditions of use. Such pharmaceutically acceptable excipients may also impart an immediate (e.g. rapid), or a modified (e.g. delayed), release of the compounds of the invention.

Suitable pharmaceutical formulations may be commercially available or otherwise are described in the literature (see, for example, Remington The Science and Practice of Pharmacy, 19th ed., Mack Printing Company, Easton, Pennsylvania (1995) and Martindale - The Complete Drug Reference (35th Edition), and the documents referred to therein). Otherwise, the preparation of suitable formulations may be achieved non-inventively by the skilled person using routine techniques. Suitable pharmaceutical formulations for use with the compounds of the invention are also described in US patent application 2010/0227812.

Accordingly, in a third aspect of the invention, there is provided a pharmaceutical composition comprising *N*,*N*-*bis*-2-mercaptoethyl isophthalamide, or a pharmaceutically-acceptable salt thereof, and optionally one or more pharmaceutically-acceptable excipient, for use in:
(a) the prevention of paracetamol toxicity (as described herein); or
(b) the prevention of acute liver failure associated with paracetamol toxicity (as described herein).

In an alternative third aspect of the invention, there is provided the use of NBMI in a method for:
(a) the prevention of paracetamol toxicity (as described herein); or
(b) the prevention of acute liver failure associated with paracetamol toxicity (as described herein),
which method comprises the administration of an effective amount of a pharmaceutical composition comprising *N*,*N*-*bis*-2-mercaptoethyl isophthalamide, or a pharmaceutically-acceptable salt thereof, and optionally one or more pharmaceutically-acceptable excipient, to a patient in need thereof.

For the avoidance of doubt, all embodiments and particular features described in relation to the first and second aspects of the invention (and combinations thereof) will also apply to the third aspect of the invention.

The skilled person will understand that references herein to pharmaceutically acceptable excipients include references to a pharmaceutically acceptable adjuvant, diluent and/or carrier, which adjuvants, diluents and carriers will be known to those skilled in the art (as described herein).

As described herein, pharmaceutical formulations may be prepared in a manner suitable for the desire route of administration, using techniques and materials known to those skilled in the art. In particular, pharmaceutical formulations may take the form of oral formulations or intravenous formulations (or formulations, e.g. concentrated formulations, suitable for use in the preparation of intravenous formulations).

For example, when intended for oral administration, pharmaceutical formulations comprising compounds of the invention may be provided in the form of a tablet, or an oral powder or solution, each optionally comprising suitable excipients, which may be prepared using techniques known to those skilled in the art. Similarly, when intended for intravenous (I.V.) administration, pharmaceutical formulations comprising compounds of the invention may be provided in the form of solutions suitable for I.V. administration, or as solutions suitable for the preparation of solutions suitable for I.V. administration, which may be prepared using techniques known to those skilled in the art. Similarly, when intended for rectal administration, pharmaceutical formulations comprising compounds of the invention may be provided in the form of a tablet (e.g. a suppository), or a powder or solution, each optionally comprising suitable excipients, which may be prepared using techniques known to those skilled in the art.

Depending on the patient to be treated, the route of administration and the severity of the condition (e.g. the level of the paracetamol overdose and the severity of the effects thereof), compounds of the invention may be administered at varying therapeutically effective doses (to the relevant patient in need thereof). Suitable doses may be determined by the skilled person using routine techniques, such as by routine dose titration studies and the like.

Similarly, the amount of the compounds of the invention included in the relevant pharmaceutical formulations may be determined based on the desired dosage of the compound of the invention, the ease of formulation and the route of administration (which may in turn determine the availability of the compound of the invention for systemic absorption).

Suitable doses of the compounds of the invention may include dosages (e.g. for I.V. administration) in the range of from about 0.05 to 300 mg/kg, such as from about 0.5 to about 200 mg/kg (e.g. about 1 to about 100 mg/kg, such as about 5 mg/kg or about 50 mg/kg).

In particular, such doses may be administered by I.V. administration, orally or rectally (e.g. by I.V administration), over a period of time, such as about 15 minutes, one hour or several hours (e.g. one hour). Moreover, such doses may be repeated as necessary, such as in the form of periodic, sequential infusions, which infusions may be of decreasing dose. For the avoidance of doubt, wherever the word "about" is employed herein, for example in the context of amounts (e.g. doses of active ingredients), it will be appreciated that such variables are approximate and as such may vary by ± 10%, for example ± 5% and preferably ± 2% (e.g. ± 1%) from the numbers specified herein.

For the avoidance of doubt, the skilled person will understand that dose of compounds of the invention administered (e.g. to a human) should be sufficient to effect the desired therapeutic response or preventative effect within (and over) a reasonable timeframe. For example, compounds of the invention may be provided in a form suitable for rapid (i.e. quick or immediate) release of the active ingredient(s), such as in the form of a rapidly disintegrating tablet, which tablets may be formulated using techniques and materials known to those skilled in the art.

In any event, the skilled person will be able to determine routinely the actual dosage which will be most suitable for an individual patient. While the above-mentioned dosages are exemplary of the average case, there can of course be individual instances where higher or lower dosage ranges are merited, and such are within the scope of the invention.

Administration of the compounds of the invention may be continuous (e.g. by continuous I.V. infusion) or intermittent (e.g. by bolus injection or through periodic administration of a tablet or solution, orally or rectally, such as by bolus injection or through periodic administration of a tablet or solution), or may be provided in the form of a single dose (e.g. by injection or through administration of a tablet or solution). The dosage form may also be determined by the timing and frequency of administration, and vice versa.

### Combinations and kits-of-parts

In the uses and methods described herein, the compounds of the invention may also be combined with one or more active ingredients that are potentially useful, or have been indicated for use, in the prevention of liver disorders.

Accordingly, in a fourth aspect of the invention, there is provided a pharmaceutical formulation comprising:
(a) *N*,*N*-*bis*-2-mercaptoethyl isophthalamide, or a pharmaceutically-acceptable salt thereof; and
(b) one or more other therapeutic agent that is useful in the prevention of paracetamol toxicity,
and optionally one or more pharmaceutically-acceptable excipients.

Further, in a fifth aspect of the invention, there is provided a kit-of-parts comprising components:
(A) a pharmaceutical formulation comprising *N*,*N*-*bis*-2-mercaptoethyl isophthalamide, or a pharmaceutically-acceptable salt thereof, and optionally one or more pharmaceutically-acceptable excipients; and
(B) a pharmaceutical formulation comprising one or more other therapeutic agent that is useful in the treatment or prevention of paracetamol toxicity, and optionally one or more pharmaceutically-acceptable excipients,
which components (A) and (B) are each provided in a form that is suitable for administration in conjunction with the other.

For the avoidance of doubt, all embodiments and particular features described in relation to the first to third aspects of the invention (and combinations thereof) will also apply to the fourth and fifth aspects of the invention.

The skilled person will understand that the kits-of-parts described herein may comprise more than one formulation including an appropriate quantity/dose of the compounds of the invention, and/or more than one formulation including an appropriate quantity/dose of one or more other therapeutic agent capable of treating or preventing paracetamol toxicity, in order to provide for repeat dosing. If more than one formulation (comprising either active compound) is present, such formulations may be the same, or may be different in terms of the dose of either compound, chemical composition(s) and/or physical form(s).

With respect to the kits-of-parts as described herein, by "administration in conjunction with" (and similarly "administered in conjunction with") we include that respective formulations comprising the compounds of the invention, and one or more other therapeutic agent capable of treating or preventing paracetamol toxicity, are administered, sequentially, separately or simultaneously, as part of the same medical intervention.

Therefore, in relation to the present invention, the term "administration in conjunction with" (and similarly "administered in conjunction with") includes that the two active ingredients (i.e. a compound of the invention, and one or more other therapeutic agent capable of treating or preventing paracetamol toxicity) are administered (optionally repeatedly) either together, or sufficiently closely in time, to enable a beneficial effect for the patient, that is greater, over the course of the treatment of the relevant condition, than if either a formulation comprising a compound of the invention, or a formulation comprising one or more other therapeutic agent capable of treating or preventing paracetamol toxicity, or pharmaceutically acceptable salt thereof, are administered (optionally repeatedly) alone, in the absence of the other component, over the same course of treatment. Determination of whether a combination provides a greater beneficial effect in respect of, and over the course of, prevention the relevant condition may be achieved routinely by the skilled person.

Further, in the context of the present invention, the term "in conjunction with" includes that one or other of the two formulations may be administered (optionally repeatedly) prior to, after, and/or at the same time as, administration of the other component. When used in this context, the terms "administered simultaneously" and "administered at the same time as" include that individual doses of a compound of the invention and one or more other therapeutic agent capable of treating or preventing paracetamol toxicity are administered within 24 hours (e.g. within 12 hours, 6 hours, 3 hours, 2 hours, 1 hour, 45 minutes, 30 minutes, 20 minutes or 10 minutes) of each other.

Thus, in relation to the fifth aspect of the invention, there is also provided a kit-of-parts comprising:
(I) one of components (A) or (B) as described in the fifth aspect of the invention; and
(II) instructions to use that component in combination with the other of the two components.

Suitable therapeutic agents useful in the treatment or prevention of paracetamol toxicity (e.g. for use in component (b) of the fourth aspect of the invention and component (B) of the fifth aspect of the invention, or for use in combination with the prevention described in the first aspect of the invention) include anti-oxidants and chelators, such as vitamin-E, vitamin-D, cysteine, cystine, glutathione, lipoic acid glutathione (GSH), dihydrolipoic acid (DLPA), lipoic acid (LPA), N-acetylcysteine (NAC), dimercaptopropane sulfonate (DMPS), dimercaptosuccinic acid (DMSA), ethylenediaminetetraacetic acid (EDTA), Deferoxamine (DFO), Deferasirox, Deferiprone, Trientine, D-penicillamine, ammonium tetrathiomolybdate, choline tetrathiomolybdate, and combinations thereof.

Particular such therapeutic agents that may be mentioned include N-acetylcysteine (NAC).

In particular embodiments of the fourth and fifth aspects of the invention, the pharmaceutical formulation or kit-of parts, as appropriate, may be for use in:
(a) the prevention of paracetamol toxicity (as defined herein); or
(b) the prevention of acute liver failure associated with paracetamol toxicity (as defined herein).

In further embodiments of the fourth and fifth aspects of the invention, the pharmaceutical formulation or kit-of parts, as appropriate, may be used in a method for:
(a) the prevention of paracetamol toxicity (as defined herein); or
(b) the prevention of acute liver failure associated with paracetamol toxicity (as defined herein),
wherein such methods may comprise administering a therapeutically effective amount of NBMI, or a pharmaceutically acceptable salt thereof, to a patient in need thereof (e.g. as a pharmaceutical formulation or kit-of-parts, as appropriate, containing the relevant therapeutically effective amount).

As also described herein, compounds of the invention may be particularly effective in preventing paracetamol toxicity or acute liver failure associated with paracetamol toxicity, and may therefore be useful in administration in combination with paracetamol (e.g. where such paracetamol is used in the treatment of mild to moderate pain or fever, as known to those skilled in the art), particularly in patients with greater susceptibility to paracetamol toxicity (as described herein) and/or those receiving or requiring treatment with greater doses of paracetamol than those normally used (e.g. doses of 1 g every 4 hours with a maximum of 4 g per 24 hour period).

Accordingly, in a sixth aspect of the invention, there is provided a pharmaceutical formulation comprising:
(a) *N*,*N*-*bis*-2-mercaptoethyl isophthalamide, or a pharmaceutically-acceptable salt thereof; and
(b) paracetamol,
and optionally one or more pharmaceutically-acceptable excipients.

Further, in a seventh aspect of the invention, there is provided a kit-of-parts comprising components:
(A) a pharmaceutical formulation comprising *N*,*N*-*bis*-2-mercaptoethyl isophthalamide, or a pharmaceutically-acceptable salt thereof, and optionally one or more pharmaceutically-acceptable excipients; and
(B) a pharmaceutical formulation comprising paracetamol, and optionally one or more pharmaceutically-acceptable excipients,
which components (A) and (B) are each provided in a form that is suitable for administration in conjunction with the other.

For the avoidance of doubt, embodiments and particular features described in relation to the first to fifth aspects of the invention (and combinations thereof) will also apply to the sixth and seventh aspects of the invention.

In relation to the seventh aspect of the invention, there is also provided a kit-of-parts comprising:
(I) one of components (A) or (B) as described in the seventh aspect of the invention; and
(II) instructions to use that component in combination with the other of the two components.

In particular, the pharmaceutical formulation as described in the sixth aspect of the invention and the kit-of-parts as described in the seventh aspect of the invention may be for use in (or may be used in a method for) the preventionof:
- mild to moderate pain or fever, particularly in patients with greater susceptibility to paracetamol toxicity (as described herein), and/or
- more severe pain or fever, and/or patients otherwise receiving or requiring treatment with greater doses of paracetamol than those normally used (e.g. doses greater than about 1 g every 4 hours with a maximum of about 4 g per 24 hour period; for example, up to about 8 g, such as up to about 16 g, and even up to about 24 g or up to about 32 g in any 24 hour period).

Suitable doses of other therapeutic agents referred to herein (such as NAC) will be known to those skilled in the art and include those listed for the drugs in question in the relevant medical literature, such as in Martindale - The Complete Drug Reference (35th Edition) and the documents referred to therein.

### Preparation of compounds and formulations

Compounds of the invention may be obtained commercially or may be prepared using techniques known to those skilled in the art, such as those described in the disclosures referneced herein. For example, NBMI may be prepared in accordance with the procedure described in US patent number US 6,586,600 B2.

As described herein, pharmacetical formulations (including those containing more than one active ingredient as described in the fourth aspect of the invention) may be prepared using techniques known to those skilled in the art. Similarly, a kit-of-parts (as described in the fifth aspect of the invention) may be prepared using techniques known to those skilled in the art.

According to further aspects of the invention, there is provided:
- a use of NBMI in a method of preparing a pharmaceutical formulation as described in the fourth and sixth aspects of the invention comprising bringing into admixture (i.e. into the same formulation) components (a) and (b) as described in the fourth and sixth aspects of the invention, optionally together with one or more pharmaceutically acceptable excipients; and
- a use of NBMI in a method of preparing a kit-of-parts as described in the fifth and seventh aspects of the invention comprising bringing into association components (A) and (B) as described in the fifth and seventh aspects of the invention.

For the avoidance of doubt, by bringing the components "into association with" each other, we include that components (A) and (B) of the kit-of-parts as described in the fifth and seventh aspects of the invention may be:
- provided as separate formulations (i.e. independently of each other), which are subsequently brought together for use in conjunction with each other in combination therapy; or
- packaged and presented as separate components of a "combination pack" for use in conjunction with each other in combination therapy.

When employed in the uses and methods described herein, compounds of the invention may have the advantage that, in the prevention of paracetamol toxicity or acute liver failure associated with paracetamol toxicity, may be more convenient for the physician and/or patient than, be more efficacious than, be less toxic than, have a broader range of activity than, be more potent than, produce fewer side effects than, or that it may have other useful pharmacological properties over, similar treatments or preventative measures known in the prior art.

Without wishing to be bound by theory, it is believed that the surprising activity of compounds of the invention in binding the toxic metabolite of paracetamol (NAPQI) in a 1:2 ratio, *in vitro* and, importantly, *in vivo* allows for an unexpectedly effective means for blocking its damaging effects on the liver, thus combating paracetamol toxicity by providing an effective means for prevention thereof.

In particular, it is believed that NBMI has the ability to effectively bind NAPQI *in vivo,* thus providing an effective means for directly blocking the *in vivo* effects of paracetamol toxicity (rather than acting as a precursor to glutathione, as is the case with agents such as NAC).

### Description of the Figures

Figure 1 illustrates the results of an *in vitro* study, wherein the activation of the calcium ion channel TRPA 1 by NAPQI was shown to be inhibited by the presence of NBMI.
Figures 2a and 2b illustrate the results of a clinical proof of concept study, which indicates that NBMI is likely to bind to NAPQI *in vivo.*
Figure 3 illustrates the effect of NBMI on paracetamol toxicity in a mouse study in which the paracetamol was dosed orally.
Figure 4 illustrates the effect of NBMI on paracetamol toxicity in an ALT assay in which the paracetamol was dosed by intraperitoneal injection.
Figure 5 illustrates the effect of NBMI on paracetamol toxicity in an AST assay in which the paracetamol was dosed by intraperitoneal injection.
Figure 6 illustrates levels of liver enzymes ALT and AST (units/L) in mice 24 hours after 200 mg/kg paracetamol administered i.p. with synchronised oral administration of either NBMI 680 mg/kg or vehicle. ** and *** markings indicate a high degree of statistical significance.

### Examples

The invention is further illustrated, but not limited, by the following examples.

### Example 1 - In vitro study showing that NBMI binds to NAPQI

The calcium ion channel TRPA 1 is activated by NAPQI. This activation may be determined by ratiometric calcium imaging.

HEK 293 cells expressing human TRPA1 were pre-incubated with 7 µM NAPQI and the calcium fluorophore FURA II. One experiment was run in the presence of 4 µM NBMI and the another was run in the presence of 4 µM NAC.

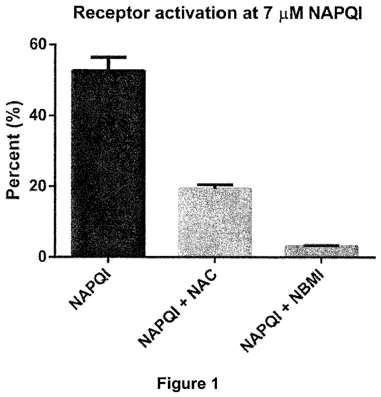

In the absence of NBMI or NAC, NAPQI caused about 55% receptor activation. As shown in Figure 1, the presence of 4 µM NBMI almost completely inhibited the activation of the receptor, whereas the presence of 4 µM NAC gave only partial inhibition.

This also indicates that every molecule of NBMI binds two NAPQI in a 1:2 relation, while NAC binds a single NAPQI in a 1:1 relation.

### Example 2 - Clinical proof of concept study

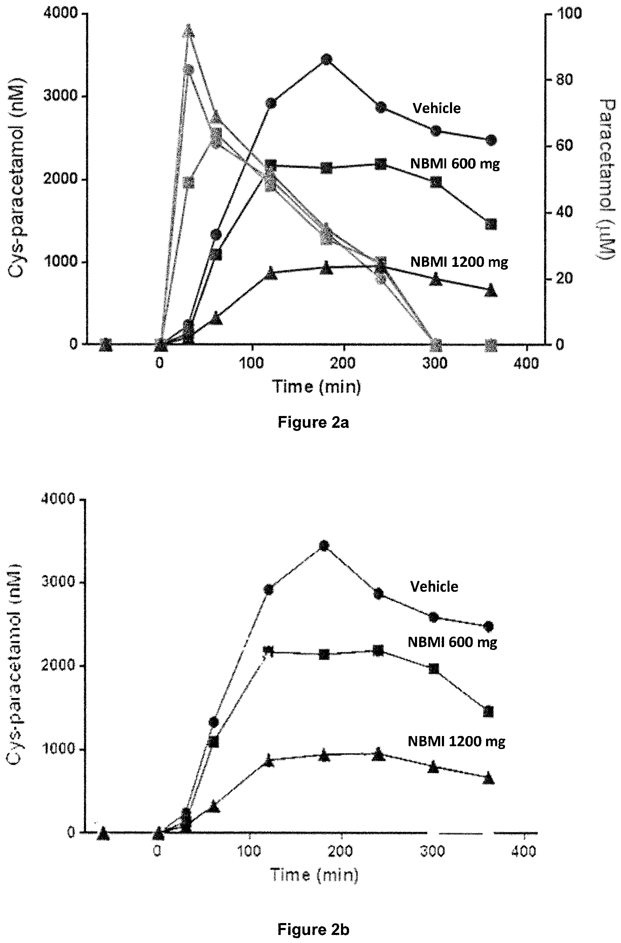

A pilot clinical study was performed at the Lund Hospital Clinical Trial Unit, on three occasions. On each occasion, a standard non-toxic therapeutic dose of 1 g paracetamol was taken at T=0 hrs with or without previous dosing of NBMI, and venous blood drawn during eight hours. The results of this experiment are summarized in Figure 2a and 2b. The three initially higher lines to the left in Figure 2a shows the concentration (uptake) of paracetamol, with the curves being almost identical (as they should be). After six hours, there is no paracetamol left, all having been broken down.

Some 5-10% of paracetamol is broken down to toxic metabolite NAPQI in the liver. To detoxify, glutathione is attached forming the metabolite glutationyl-paracetamol, which is then turned into the non-toxic metabolite cysteinyl-paracetamol (Cys-paracetamol), which can be measured in the blood, shown by the line marked "Vehicle" in Figure 2b.

The two lines marked with NBMI concentrations in Figure 2b show that the concentration of cys-paracetamol is lower when NBMI has been taken prior to paracetamol administration, showing that 600 mg NBMI taken one hour prior to paracetamol administration decreased the area under the curve (AUC) by 23%, and that 1,200 mg NBMI taken two hours before paracetamol administration decreased it by 73%.

These results indicate that NBMI binds to NAPQI in the presence of glutathione *in vivo.*

### General information - Examples 3 to 5

In normal mice, the liver enzyme alanine transaminase (ALT) level is about 36-40 unit/L and the liver enzyme aspartate transaminase (AST) level is about 90 unit/L. At toxic doses of paracetamol, increasing levels of the toxic metabolite NAPQI depletes the glutathione in the liver, resulting in oxidative stress and in ALT and/or AST levels increasing above 1,000 unit/L, which in humans is considered toxic. The ratio of AST:ALT can also be relevant in humans; a value at or below 1 is considered to indicate toxicity.

### Example 3 - Paracetamol intoxicated mice study (oral administration of paracetamol)

### Study design

Six (6) mice, fasted overnight, each received a toxic dose 700 mg/kg paracetamol (acetaminophen) per os. 20 minutes later, half of the mice (3) were administered vehicle (0.5 % (w/v) carboxymethylcellulose 300-600 centipoises in water) only, half of the mice (3) received NBMI 100 mg/kg per os (NBMI dissolved in CMC as described below). All mice are sacrificed using anesthesia after 48 hours or earlier if required.

### Results

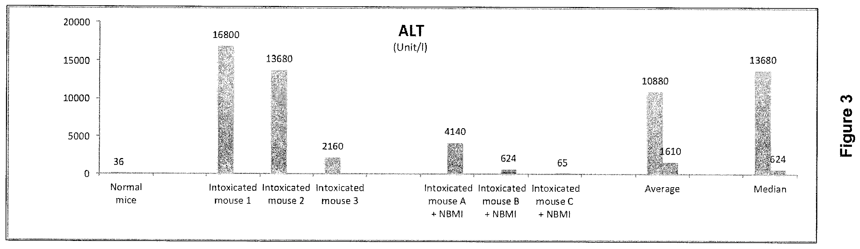

The mice needed to be sacrificed at 17 hours as the intoxicated (i.e. paracetamol intoxicated) control mice were in poor condition. The results are summarized in Figure 3, which shows the liver enzyme ALT levels as unit/L of the mice

The median ALT level in control mice is 13,680 Unit/L, approximately 380 times higher than normal levels (36 unit/L), and 13 times the toxic level in humans (1,000 unit/L).

The median ALT level in the NBMI treated group is 624 unit/L, thus about 95% lower than the median of the intoxicated animals, and under the toxic level for humans.

### Example 4 - Paracetamol intoxicated mice study (i.p. administration of paracetamol)

### Study design

Six (6) mice received a toxic dose of 300 mg/kg paracetamol (acetaminophen; APAP) intraperitoneally (I.P.). 30 minutes later, two (2) of the mice were administered vehicle only, two (2) received NAC 1200 mg/kg per os, two (2) received NBMI 200 mg/kg per os. The mice were sacrificed at 24 hours.

### Results ALT

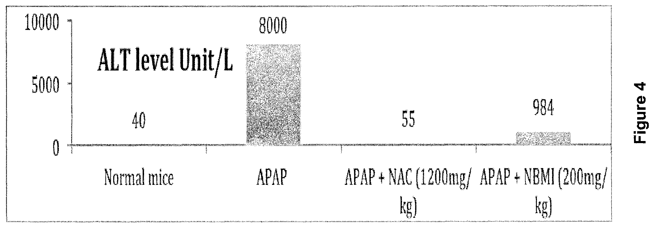

The results in the ALT assay are shown in Figure 4. It can be seen that the intoxicated mice had ALT levels of approximately 8,000 unit/L. Administration of NAC kept ALT at an average level of 55. Administration of NBMI at 200 mg/kg kept ALT at an average level of 984, which is below the intoxication level of 1,000.

### Results AST

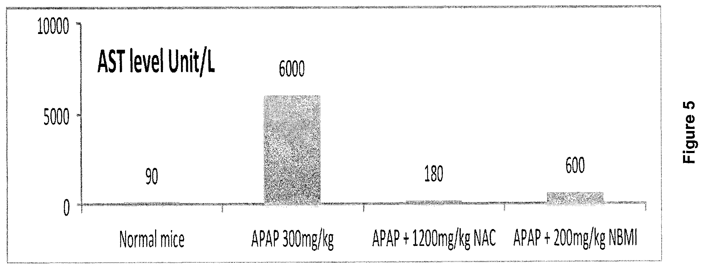

The results of the AST assay are shown in Figure 5. The intoxicated mice had AST levels of approximately 6,000 unit/L. Administration of NAC kept AST at 180. Administration of NBMI at 200 mg/kg kept the ALT level at 600.

### Example 5 - Effect of NMBI administration compared to control (vehicle only) in paracetamol intoxicated mice (i.p. administration of paracetamol)

Mice that had fasted were injected i.p. with an 200mg/kg paracetamol, and synchroneously with either an oral dose of either (i) 680 mg/kg NBMI in CMC vehicle (carboxymethylcellulose sodium salt of medium viscosity), or (ii) CMC vehicle alone; dosing volume 5µL/gram body weight. The NBMI dose was chosen as it has in 28-day-toxicity studies shown to provide a similar exposure as a 4.5-9 mg/kg dose in humans, a dose showing no drug related adverse events in clinical trials. Mice were sacrificed at 24 hours after administration, following which levels of liver enzymes AST and ALT were measured.

In the NBMI-treated group, eleven (11) mice were treated. One result was discounted as an outlier (based on Grubbs' test for outliers, and due to hemolysis having been observed). An average of the results obtained from the remaining mice showed:
AST (units/L) - 753 (P-value = 0.00204, as calculated by Excel)
ALT (units/L) - 952 (P-value = 0.00006, as calculated by Excel)
AST/ALT ratio - 2.1 (P-value = 0.00068, as calculated by Excel)

In the vehicle-only group, thirteen mice were treated. An average of the results obtained showed:
AST (units/L) - 8507
ALT (units/L) - 14511
AST/ALT ratio - 0.5

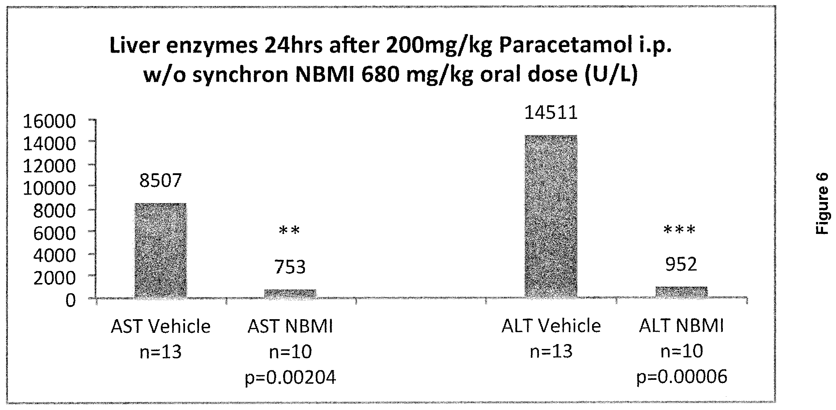

The results are summarized in Figure 6.

## Claims

1. *N*,*N*-*bis*-2-mercaptoethyl isophthalamide (NBMI), or a pharmaceutically-acceptable salt thereof, for use in the prevention of paracetamol toxicity.

2. *N*,*N*-*bis*-2-mercaptoethyl isophthalamide (NBMI), or a pharmaceutically-acceptable salt thereof, for use in the prevention of acute liver failure associated with paracetamol toxicity.

3. A pharmaceutical formulation comprising:
(a) *N*,*N*-*bis*-2-mercaptoethyl isophthalamide (NBMI), or a pharmaceutically-acceptable salt thereof; and
(b) paracetamol,
and optionally one or more pharmaceutically-acceptable excipients.

4. A kit-of-parts comprising components:
(A) a pharmaceutical formulation comprising *N*,*N*-*bis*-2-mercaptoethyl isophthalamide (NBMI), or a pharmaceutically-acceptable salt thereof, and optionally one or more pharmaceutically-acceptable excipients; and
(B) a pharmaceutical formulation comprising paracetamol, and optionally one or more pharmaceutically-acceptable excipients,
which components (A) and (B) are each provided in a form that is suitable for administration in conjunction with the other.

## Patentansprüche

1. *N*,*N*-Bis-2-mercaptoethylisophthalamid (NBMI) oder ein pharmazeutisch unbedenkliches Salz davon zur Verwendung bei der Vorbeugung von Paracetamol-Toxizität.

2. *N*,*N*-Bis-2-mercaptoethylisophthalamid (NBMI) oder ein pharmazeutisch unbedenkliches Salz davon zur Verwendung bei der Vorbeugung von akutem Leberversagen, das mit Paracetamol-Toxizität verknüpft ist.

3. Pharmazeutische Formulierung, die Folgendes umfasst:
(a) *N*,*N*-Bis-2-mercaptoethylisophthalamid (NBMI) oder ein pharmazeutisch unbedenkliches Salz davon; und
(b) Paracetamol,
und optional einen oder mehrere pharmazeutisch unbedenkliche Arzneistoffträger.

4. Bausatz, der folgende Komponenten umfasst:
(A) eine pharmazeutische Formulierung, die *N*,*N*-Bis-2-mercaptoethylisophthalamid (NBMI) oder ein pharmazeutisch unbedenkliches Salz davon und optional einen oder mehrere pharmazeutisch unbedenkliche Arzneistoffträger umfasst; und
(B) eine pharmazeutische Formulierung, die Paracetamol und optional einen oder mehrere pharmazeutisch unbedenkliche Arzneistoffträger umfasst,
wobei Komponente (a) und (b) jeweils in einer Form bereitgestellt sind, die für eine Verabreichung in Verbindung mit der anderen geeignet ist.

## Revendications

1. *N*,*N*-bis-2-mercaptoéthyl isophtalamide (NBMI), ou sel pharmaceutiquement acceptable de celui-ci, destiné à être utilisé dans la prévention de la toxicité du paracétamol.

2. *N*,*N*-bis-2-mercaptoéthyl isophtalamide (NBMI), ou sel pharmaceutiquement acceptable de celui-ci, destiné à être utilisé dans la prévention de l'insuffisance hépatique aiguë associée à la toxicité du paracétamol.

3. Formulation pharmaceutique comprenant :
(a) du *N*,*N*-bis-2-mercaptoéthyl isophtalamide (NBMI), ou un sel pharmaceutiquement acceptable de celui-ci ; et
(b) du paracétamol,
et éventuellement un ou plusieurs excipients pharmaceutiquement acceptables.

4. Kit d'éléments comprenant des composants :
(A) une formulation pharmaceutique comprenant du *N*,*N*-bis-2-mercaptoéthyl isophtalamide (NBMI), ou un sel pharmaceutiquement acceptable de celui-ci, et éventuellement un ou plusieurs excipients pharmaceutiquement acceptables ; et (B) une formulation pharmaceutique comprenant du paracétamol, et éventuellement un ou plusieurs excipients pharmaceutiquement acceptables,
lesquels composants (A) et (B) étant chacun fournis sous une forme qui est appropriée pour une administration conjointement avec l'autre.
